# EUROPÄISCHE PATENTSCHRIFT

(11) **EP 1 319 714 B1**
(45) Veröffentlichungstag und Bekanntmachung des Hinweises auf die Patenterteilung: **01.06.2005**
(21) Anmeldenummer: 02027110.2
(22) Anmeldetag: 04.12.2002
(51) Int. Cl.: C12P 7/40, C12P 7/64

(54) **Verfahren zur Herstellung von C4-C12-Fettsäuren**
Process for the production of C4-C12 fatty acids
Procédé de production d'acides gras C4-C12

(30) Priorität: 13.12.2001 DE 10161274
(43) Veröffentlichungstag der Anmeldung: 18.06.2003
(73) Patentinhaber: Cognis Deutschland GmbH & Co. KG, 40589 Düsseldorf (DE)
(72) Erfinder: Yüksel, Levent, Dr., 40597 Düsseldorf (DE); Fieg, Georg, Dr., 40822 Mettmann (DE); Otto, Ralf, Dr., 74177 Bad Friedrichshall (DE); Schörken, Ulrich, Dr., 40591 Düsseldorf (DE); Both, Sabine, Dr., 40229 Düsseldorf (DE); Mrozek, Ingomar, Dr., 40237 Düsseldorf (DE)

(56) Entgegenhaltungen:
- EP-A- 0 257 716
- WO-A-03/010323
- US-A- 3 926 726
- US-A- 5 032 515
- US-A- 5 830 719
- BUEHLER M ET AL: "Continuous use of lipases in fat hydrolysis" FETT - LIPID, WILEY-VCH VERLAG,WEINHEIM, DE, Bd. 89, Nr. 14, 1987, Seiten 598-605, XP002188305 ISSN: 0931-5985
- BUEHLER M ET AL: "CONTINUOUS USE OF LIPASES IN FAT HYDROLYSIS" JOURNAL OF THE AMERICAN OIL CHEMISTS' SOCIETY, Bd. 64, Nr. 9, 1987, Seiten 1257-1258, XP001148819 ISSN: 0003-021X
- GAN Q ET AL: "SIMULTANEOUS REACTION AND SEPARATION IN ENZYMATIC HYDROLYSIS OF HIGH OLEATE SUNFLOWER OIL - EVALUATION OF ULTRAFILTRATION PERFORMANCE AND PROCESS SYNERGY" CHEMICAL ENGINEERING JOURNAL, ELSEVIER SEQUOIA, LAUSANNE, CH, Bd. 71, Nr. 2, 21. Dezember 1998 (1998-12-21), Seiten 87-96, XP001022886 ISSN: 1385-8947

## Beschreibung

### Gebiet der Erfindung

Die Erfindung befindet sich auf dem Gebiet der oleochemischen Rohstoffe und betrifft ein biotechnologisches Verfahren zur Herstellung von kurzkettigen Fettsäuren aus den entsprechenden Methylestern.

### Stand der Technik

In der Oleochemie werden Fettsäuremethylester mit unterschiedlicher Kettenlängenverteilung produziert. Bei der Abtrennung von längerkettigen Fettsäuremethylestern durch Destillation entstehen sogenannte Vorlauf-Fettsäuremethylester, bei denen es sich um unterschiedlichste Gemische von C₄- bis C₁₂-Methylestern handelt und die vielfach direkt in weitere Umesterungsreaktionen eingesetzt werden. Die daraus resultierenden Derivate sind allerdings aufgrund des unreinen Rohstoffs von schlechter Qualität. Alternativ werden daher die Fettsäuremethylester zunächst gespalten und die freigesetzten Fettsäuren dann verestert. Die chemische Hydrolyse erfolgt in Gegenwart saurer Katalysatoren, wie beispielsweise Alkylbenzolsulfonsäuren. Im Verfahren kommt es daher zur Bildung von Schwefelsäure, die in den Anlagen zu massiver Korrosion führt und die Produkte mit hohen Metallgehalten verunreinigt. Ein weiteres Problem besteht in der umweltgerechten Entsorgung der Katalysatoren.

Die Aufgabe der vorliegenden Erfindung hat somit darin bestanden, ein verbessertes Verfahren zur Herstellung von kurzkettigen Fettsäuren aus deren Methylestern zur Verfügung zu stellen, welches die genannten Nachteile des Stands der Technik zuverlässig vermeidet. Insbesondere sollten die Fettsäuren in hoher Reinheit und hohen Ausbeuten erhalten werden und das Verfahren unter milden Bedingungen arbeiten.

### Beschreibung der Erfindung

Gegenstand der Erfindung ist ein Verfahren zur Herstellung von C₄-C₁₂-Fettsäuren, bei dem man
(a) C₄-C₁₂-Fettsäuremethylester in Gegenwart von Lipasen mit Wasser zu einem ersten Hydrolysat spaltet,
(b) das erste Hydrolysat in eine organische und eine wässrig/alkoholische Phase auftrennt,
(c) die organische Phase, enthaltend Fettsäuren und Fettsäuremethylester in Gegenwart einer weiteren Menge an Lipasen zu einem zweiten Hydrolysat spaltet
(d) das zweite Hydrolysat wiederum in eine organische und eine wässrig/alkoholische Phase auftrennt,
(e) und die zweite organische Phase, enthaltend Fettsäuren und Fettsäuremethylester von nicht-umgesetzten Fettsäuremethylestern befreit.

Überraschenderweise wurde gefunden, dass die zweistufige enzymatische Hydrolyse zu Fettsäuren führt, die frei von unerwünschten Nebenprodukten führt. Es werden hohe Ausbeuten erzielt, das Verfahren arbeitet bei milden Bedingungen und mit Katalysatoren, die alle Anforderungen an die Umweltverträglichkeit erfüllen.

### Hydrolyse

Die Hydrolyse der Fettsäuremethylester erfolgt vorzugsweise bei milden Temperaturen im Bereich von 20 bis 50 und vorzugsweise 30 bis 40 °C, wo bei die bevorzugte Temperatur durch das Aktivitätsoptimum der eingesetzten Lipase vorgegeben wird. Üblicherweise werden die Lipasen in Mengen von 0,001 bis 1 und vorzugsweise 0,01 bis 0,1 Gew.-% bezogen auf die Fettsäuremethylester eingesetzt. Typische Beispiele für geeignete Lipasen sind die Handelsprodukte Novozym 388 L, Novozym 525 L, Lipozym TL 100 und Amano G. Die Enzyme werden in der Regel als verdünnte Suspensionen oder wässrige Konzentrate eingesetzt. Es ist jedoch auch möglich, die Reaktion in Reaktoren statt mit freier, nativer Lipase auch mit immobilisierten Enzymen durchzuführen. Die immobilisierte Lipase kann entweder adsorptiv oder kovalent an einen Träger gebunden sein. In diesem Fall werden die Ausgangsstoffe über ein Katalysatorfestbett gefahren. Es hat sich als vorteilhaft erwiesen, die Hydrolyse in zwei Schritten durchzuführen und zwischen der ersten und zweiten Hydrolyse einen Phasenwechsel durchzuführen, d.h. die organische Phase abzutrennen und mit frischem Enzym zu versetzen. Vorzugsweise betreibt man den ersten Hydrolyseschritt bis zu einem Spaltgrad von 40 bis 60, vorzugsweise 50 bis 55 Gew.-% und die zweite Hydrolyse bis zu einem Spaltgrad von 60 bis 80, vorzugsweise 70 bis 75 Gew.-%. Der Phasenwechsel bedeutet die Auftrennung des Hydrolysats in eine wässrig/alkoholische Phase, die Wasser, durch Spaltung gebildetes Methanol sowie die Enzyme enthält, sowie eine organische Phase, in der sich die gebildeten Fettsäuren sowie nicht umgesetztes Ausgangsmaterial befinden. Die Trennung kann nach den Verfahren des Stands der Technik erfolgen, bevorzugt ist aber die Trennung durch Zentrifugation. Alternativ ist es auch möglich, das bei der Hydrolyse entstehende Methanol kontinuierlich, beispielsweise durch Destillation aus der Reaktion abzutrennen. Dies kann beispielsweise einem Hydrolysereaktor mit aufgesetztem Verdampfer und Methanolkondensation erfolgen.

### Aufarbeitung

Im Anschluss an die zweite Hydrolyse wird wiederum die wässrig/alkoholische von der organischen Phase getrennt und letztere aufgearbeitet, d.h. nicht umgesetzter Methylester vom Wertprodukt entfernt. Dies erfolgt vorzugsweise in einer Destillationskolonne mit gepackten Einbauten, wobei es sich als vorteilhaft erwiesen hat, den Feed zwischen Auftriebs- und Abtriebsteil der Kolonne einzuspeisen. Bei Temperaturen im Bereich von 70 bis 100 °C und einem verminderten Druck von 10 bis 50 mbar werden die Methylester am Kopf der Kolonne abgezogen und können in die Reaktion zurückgeführt werden. Kürzerkettige Fettsäuren sowie niedrig siedende Verunreinigungen können über die Pumpe abgesaugt und in die Abluft gelangen, weswegen sich eine nachgeschaltete Kondensation empfiehlt. Die resultierenden Fettsäuren weisen eine Reinheit größer 95 Gew.-% auf.

### Beispiele

**Beispiel 1.** In einer Rührapparatur mit einem Fassungsvermögen von 3 I wurden 540 g eines C₈-Vorlauffettsäuremethylesters vorgelegt, mit 1260 g Wasser und 16.2 ml Lipolase-Konzentrat versetzt und bei 37 °C mit einer Geschwindigkeit von 300 Upm gerührt. Durch Probennahme wurde die Hydrolyse verfolgt. Nach 16 h wurde die Hydrolyse abgebrochen, das so erhaltene erste Hydrolysat in eine Zentrifuge überführt und in eine wässrig/alkoholische Phase (enthaltend Wasser, Methanol und Enzyme) und eine organische Phase getrennt, welche die gebildeten Fettsäure und den noch nicht umgesetzten Methylester enthielt, getrennt. Die organische Phase wurde in den Reaktor zurückgeführt, mit 1260 g Wasser und 16,2 ml frischem Enzym versetzt. Anschließend wurde die Mischung einer zweiten Hydrolyse wiederum bei 37 °C unterworfen. Auch hier wurde der Reaktionsfortschritt durch Probennahme verfolgt. Die Ergebnisse sind in Tabelle 1 zusammengefasst.

**Tabelle 1**

| **Hydrolyse von Vorlauffettsäuremethylestern (Angaben in Gew.-%)** | | | | |
|---|---|---|---|---|
| **Hydrolysedauer** **h** | **1. Hydrolyse** | | **2. Hydrolyse** | |
| | Methylester | Fettsäure | Methylester | Fettsäure |
| 0,25 | 80,2 | 18,0 | | |
| 0,5 | | | 46,2 | 51,8 |
| 0,75 | 61,1 | 36,9 | | |
| 1,0 | 57,6 | 40,4 | | |
| 1,5 | 49,6 | 48,3 | 42,5 | 55,5 |
| 2,5 | | | 40,5 | 57,5 |
| 4,5 | | | 37,2 | 60,8 |
| 16 | 45,0 | 53,0 | | |
| 20 | | | 30,8 | 67,1 |

Das zweite Hydrolysat, welches 67,1 Fettsäure und 30,8 Gew.-% nicht umgesetzten Methylester enthielt, wurde wiederum durch Zentrifugation in eine wässrige/alkoholische und eine organische Phase aufgetrennt. Letztere wurde zwischen Auftriebs- und Abtriebsteil in eine Rektifikationskolonne mit gepackten Einbauten aufgegeben und bei 85 °C und 20 mbar destilliert. Nach 6 h, während der kürzerkettige und niedrigsiedende Verunreinigungen über die Pumpe abgesaugt wurden, wurde eine C₈-Fettsäure mit einer Reinheit von größer 95 Gew.-% erhalten.

**Beispiel 2.** Analog Beispiel 1 wurde die Aktivität verschiedener Lipasen in der Hydrolyse getestet und Proben nach jeweils 24 und 48 h gezogen. Eingesetzt wurden 3 g C₈-Vorlauffettsäuremethylester, 7 g Wasser und jeweils 1g/1ml Lipase (10 Gew.-% Feststoffgehalt). Die Ergebnisse sind in Tabelle 2 zusammengefasst.

**Tabelle 2**

| **Aktivität verschiedener Lipasen in der Hydrolyse (Angaben in Gew.-%)** | | | | |
|---|---|---|---|---|
| **Lipase** | **24 h** | | **48 h** | |
| | Methylester | Fettsäure | Methylester | Fettsäure |
| Novozym 388 L | 49,5 | 48,8 | 27,8 | 70,6 |
| Novozym 525 L | 48,8 | 49,6 | 28,6 | 69,8 |
| Lipozym TL 100 | 49,2 | 48,6 | 26,1 | 70,1 |
| Amano G | 50,9 | 47,7 | 26,3 | 72,2 |

**Beispiel 3.** Analog Beispiel 1 wurde die Aktivität verschiedener Lipasen in der Hydrolyse getestet und Proben nach jeweils 2 und 4 h gezogen. Eingesetzt wurden 6 g C₈-Vorlauffettsäuremethylester, 14 g Wasser und jeweils 1ml Lipase (10 Gew.-% Feststoffgehalt). Nach 2 h erfolgte ein Austausch der Phasen und erneute Zugabe von Enzym. Die Ergebnisse sind in Tabelle 3 zusammengefasst.

**Tabelle 3**

| **Aktivität verschiedener Lipasen in der Hydrolyse (Angaben in Gew.-%)** | | | | |
|---|---|---|---|---|
| **Lipase** | **2 h** | | **4 h** | |
| | Methylester | Fettsäure | Methylester | Fettsäure |
| Novozym 525 L | 45,2 | 53,0 | 9,5 | 89,0 |
| Lipozym TL 100 | 43,5 | 54,3 | 15,5 | 79,0 |

## Patentansprüche

1. Verfahren zur Herstellung von C₄-C₁₂-Fettsäuren, bei dem man
(a) C₄-C₁₂-Fettsäuremethylester in Gegenwart von Lipasen mit Wasser zu einem ersten Hydrolysat spaltet und die erste Hydrolyse bis zu einem Spaltgrad von 40 bis 60 Gew.-% durchführt,
(b) das erste Hydrolysat in eine organische und eine wässrig/alkoholische Phase enthaltend Wasser, durch Spaltung gebildetes Methanol sowie die Enzyme auftrennt,
(c) die organische Phase, enthaltend Fettsäuren und Fettsäuremethylester in Gegenwart einer weiteren Menge an Lipasen zu einem zweiten Hydrolysat spaltet
(d) das zweite Hydrolysat wiederum in eine organische und eine wässrig/alkoholische Phase auftrennt,
(e) und die zweite organische Phase, enthaltend Fettsäuren und Fettsäuremethylester von nicht-umgesetzten Fettsäuremethylestern befreit.

2. Verfahren nach Anspruch 1, **dadurch gekennzeichnet, dass** man die erste Hydrolysen bei Temperaturen im Bereich von 20 bis 50 °C einsetzt.

3. Verfahren nach den Ansprüchen 1 und/oder 2, **dadurch gekennzeichnet, dass** man die Lipasen in Mengen von 0,001 bis 1 Gew.-% bezogen auf die Fettsäuremethylester einsetzt.

4. Verfahren nach mindestens einem der Ansprüche 1 bis 3, **dadurch gekennzeichnet, dass** man freie native Lipasen und/oder immobilisierte Lipasen einsetzt.

5. Verfahren nach mindestens einem der Ansprüche 1 bis 4, **dadurch gekennzeichnet, dass** man die zweite Hydrolyse bis zu einem Spaltgrad von 60 bis 80 Gew.-% durchführt.

6. Verfahren nach mindestens einem der Ansprüche 1 bis 5, **dadurch gekennzeichnet, dass** man die organische und wässrig/alkoholische Phase durch Zentrifugieren trennt.

## Claims

1. A process for the production of C₄-C₁₂ fatty acids, in which
(a) C₄-C₁₂ fatty acid methyl esters are hydrolyzed with water in the presence of lipases to form a first hydrolyzate and the first hydrolysis is carried out to a hydrolysis level of 40 to 60% by weight,
(b) the first hydrolyzate is separated into an organic phase and an aqueous/alcoholic phase containing water, methanol formed by hydrolysis and the enzymes,
(c) the organic phase containing fatty acids and fatty acid methyl esters is hydrolyzed in the presence of more lipases to form a second hydrolyzate,
(d) the second hydrolyzate is separated into an organic phase and an aqueous/alcoholic phase,
(e) and the second organic phase containing fatty acids and fatty acid methyl esters is freed from unreacted fatty acid methyl esters.

2. A process as claimed in claim 1, **characterized in that** the first hydrolysis is carried out at temperatures in the range from 20 to 50°C.

3. A process as claimed in claims 1 and/or 2, **characterized in that** the lipases are used in quantities of 0.001 to 1% by weight, based on the fatty acid methyl esters.

4. A process as claimed in at least one of claims 1 to 3, **characterized in that** free native lipases and/or immobilized lipases are used.

5. A process as claimed in at least one of claims 1 to 4, **characterized in that** the second hydrolysis is carried out to a hydrolysis level of 60 to 80% by weight.

6. A process as claimed in at least one of claims 1 to 5, **characterized in that** the organic phase and the aqueous/alcoholic phase are separated by centrifuging.

## Revendications

1. Procédé pour la préparation d'acides gras en C₄-C₁₂, selon lequel
(a) on décompose des esters méthyliques d'acides gras en C₄-C₁₂ avec de l'eau en présence de lipases, pour obtenir un premier hydrolysat et on effectue la première hydrolyse jusqu'à un degré de décomposition de 40 à 60 % en poids,
(b) on fractionne le premier hydrolysat en une phase organique et une phase aqueuse/alcoolique contenant de l'eau par décomposition du méthanol formé ainsi que les enzymes,
(c) on décompose la phase organique, contenant des acides gras et des esters méthyliques d'acides gras, en présence d'une nouvelle quantité de lipases, pour obtenir un second hydrolysat,
(d) on fractionne à son tour le second hydrolysat en une phase organique et une phase aqueuse/alcoolique,
(e) et on sépare les esters méthyliques d'acides gras n'ayant pas réagi de la seconde phase organique contenant des acides gras et des esters méthyliques d'acides gras.

2. Procédé selon la revendication 1,
**caractérisé en ce qu'**
on met en oeuvre la première hydrolyse à des températures dans la plage de 20 à 50°C.

3. Procédé selon la revendication 1 et/ou la revendication 2,
**caractérisé en ce qu'**
on utilise les lipases en proportions de 0,001 à 1 % en poids, par rapport aux esters méthyliques d'acides gras.

4. Procédé selon au moins une des revendications 1 à 3,
**caractérisé en ce qu'**
on utilise des lipases natives libres et/ou des lipases immobilisées.

5. Procédé selon au moins une des revendications 1 à 4,
**caractérisé en ce qu'**
on effectue la seconde hydrolyse jusqu'à un degré de décomposition de 60 à 80 %.

6. Procédé selon au moins une des revendications 1 à 5,
**caractérisé en ce qu'**
on sépare la phase organique et la phase aqueuse/alcoolique par centrifugation.
